# DEMANDE DE BREVET EUROPEEN

(11) **EP 4 218 578 A1**
(43) Date de publication de la demande: **02.08.2023**
(21) Numéro de dépôt: 23166128.1
(22) Date de dépôt: 22.07.2022
(51) Int. Cl.: A61B 5/282, A61B 5/332, A61B 5/024, A61B 5/0245, G04G 21/02, A61B 5/00, G04G 17/04, G04G 17/08

(54) **MONTRE CONNECTÉE AVEC LUNETTE ROTATIVE**

(30) Priorité: 10.08.2021 FR 2108606
(62) Demande divisionnaire de: 22186501.7
(71) Demandeur: Withings, 92130 Issy-Les-Moulineaux (FR)
(72) Inventeur: Donnet, Pierre-Arnaud, 92130 Issy-les-Moulineaux (FR); Clerc, Samuel, 92130 Issy-les-Moulineaux (FR); Navellou, Manon, 92130 Issy-les-Moulineaux (FR)
(74) Mandataire: Withings IP

(57) **Abrégé**

Il est proposé une montre connectée de type « DIVER », avec une lunette (bezel) rotative. La montre comprend un module électronique configuré pour effectuer un électrocardiogramme (ECG ou EKG) à l'aide d'au moins deux électrodes intégrées à la montre. Une des deux électrodes est formée par la lunette rotative.

## Description

### Domaine technique

La présente invention concerne le domaine des montres connectées et en particulier des montres connectées capables d'effectuer un électrocardiogramme (ECG ou EKG). Par montre, il est signifié un dispositif portable (« *wearable* » en anglais), dont la position préférentielle est le poignet (« *wrist wearable* »). La présente invention concerne aussi le domaine des montres connectées dites hybrides, c'est-à-dire des montres connectées présentant un aspect visuel plus proche des montres mécaniques conventionnelles, notamment grâce à la présence d'un train d'engrenages ainsi que d'aiguilles mécaniques pour indiquer au moins l'heure (aiguille des heures et aiguille des minutes).

### Technique antérieure

Les montres permettant d'effectuer un ECG (appelées « montre-ECG » par la suite) sont peu nombreuses sur le marché en 2021. On peut notamment citer la Withings Move ECG, la Withings ScanWatch, l'Apple Watch et la Samsung Galaxy.

Un ECG est un test qui permet d'étudier le fonctionnement du coeur en mesurant son activité électrique. Une impulsion électrique traverse le coeur à chaque contraction et l'ECG constitue un tracé obtenu en enregistrant les courants électriques. L'ECG fait partie des mesures principales pour le suivi cardiovasculaire. Grâce à son intégration dans une montre, tout utilisateur peut régulièrement effectuer un ECG, ce qui permet une meilleure analyse et prévention des risques cardiaques. Pour effectuer un ECG, plusieurs électrodes sont positionnées sur le corps humain, afin de détecter différentes composantes des signaux électriques qui sont appelées dérivations ou voies (« *leads »* en anglais). Dans une des formes les plus simples, l'ECG permet d'obtenir une mesure bipolaire entre bras droit et bras gauche, appelée dérivation | ou DI.

Le test est indolore, passif et non invasif (pas d'injection de courant dans la peau) et peut être effectué en moins d'une minute. Bien que le concept d'intégrer un ECG dans un dispositif grand public ait été présenté depuis plusieurs années (voir US5289824), l'implémentation effective n'a été réussie que récemment. Il s'agit d'une innovation technologique majeure.

Les montres-ECG permettent généralement d'obtenir un ECG DI, même si d'autres dérivations peuvent être obtenues. A cette fin, une montre-ECG comprend une carrure (« *case* » en anglais), un fond de carrure (« *case back* »), une glace (« *glass* »), et soit un cadran (avec éventuellement un écran ou un affichage numérique), soit un écran. On peut distinguer les montres-ECG de type smartwatch et les montres-ECG de type hybride. Dans le premier cas, la montre-ECG comprend un écran (par exemple un écran LCD) et la glace, en lieu et place du cadran et de la glace de montre traditionnelle. Dans le deuxième cas, la montre-ECG comprend un cadran, avec des aiguilles et la glace (avec ou sans écran intégré au cadran), donnant ainsi un aspect plus traditionnel à l'ensemble. Les montres-ECG peuvent aussi comprendre une lunette (« *bezel* » en anglais), qui est une pièce entourant l'écran ou le cadran (et/ou le verre). De la même façon, les montres-ECG peuvent comprendre une couronne (« *crown* » en anglais), qui traverse radialement la carrure (typiquement positionnée entre 1h et 5h) pour pénétrer dans un volume défini par celui-ci, et qui permet d'interagir avec des composants électroniques ou mécaniques de la montre (activation de menu, défilement, etc.). La couronne peut être un bouton-pression (« *pusher* ») et/ou être une molette rotative.

Pour réaliser un ECG avec une montre, au moins deux électrodes (souvent trois) sont nécessaires. Elles doivent être en contact avec deux portions différentes du corps. Le document US2019246979A1 propose d'intégrer les deux électrodes au bracelet une sur la face interne et une sur la face externe. D'autres documents proposent de monter une première électrode sur le fond de carrure, afin d'être en contact avec le poignet sur lequel la montre-ECG est montée. Le choix de la deuxième électrode a fait l'objet de différentes orientations technologiques. Le document US2020229761, au nom d'Apple, propose d'intégrer la deuxième électrode dans la couronne. Le document PCT/EP2021/058955, au nom de Withings (et incorporé ici par référence) et non publié le jour du dépôt de la présente demande, propose intégrer la deuxième électrode dans la lunette. D'autres solutions existent, comme une deuxième électrode positionnée sur l'écran ou à côté de l'écran.

L'implémentation d'un dispositif de mesure d'ECG dans une montre présente de nombreuses difficultés techniques, notamment en ce que les signaux électriques à identifier sont de faibles amplitudes et en ce que leur acquisition est fréquemment bruitée. Le bon positionnement des électrodes et la gestion de la chaîne électrique entre les électrodes et le module électronique qui gère l'ECG sont cruciaux.

### Résumé

Dans une volonté à la fois de diversifier les gammes et de rendre les montres connectées le plus accessibles possible en matière de design et d'utilisation, il a été souhaité par les inventeurs de fabriquer un modèle respectant autant que possible les codes esthétiques des montres de type DIVER, en particulier (dans le cadre de la présente description) d'une montre connectée présentant une lunette rotative. Les avantages d'une utilisation généralisée d'un montre-ECG sont multiples : la prévention des risques cardiaques est améliorée, le coût financier sur la société est allégé et l'état des connaissances scientifiques est démultiplié en donnant accès à des données régulières d'ECG sur des milliers ou des millions de gens (par rapport des tests ponctuels en hôpital ou en laboratoire). De plus, les montres-ECG ont généralement d'autres fonctions (par exemple la mesure du taux de saturation en oxygène SpO2 ou du rythme cardiaque par PPG), de sorte que ces montres présentent un intérêt supérieur pour la recherche en permettant de combiner les données.

La présente invention concerne un dispositif électronique portatif, configuré pour être positionné sur un poignet, le dispositif portatif permettant d'effectuer un électrocardiogramme, ECG, ledit dispositif comportant :
- une carrure,
- un fond de carrure, configuré pour être au moins partiellement en contact avec la peau du poignet,
- une glace,
- une lunette, montée sur la carrure et entourant la glace, mobile en rotation par rapport à la carrure,
- une première électrode ECG, en matériau conducteur, sur le fond de carrure et configurée pour être en contact avec la peau du poignet,
- une deuxième électrode ECG, en matériau conducteur, sur la lunette,
- un module électronique ECG, électriquement connecté à la première électrode ECG et la deuxième électrode ECG, et configuré pour recevoir et traiter des signaux électriques venant d'un utilisateur et récupérés par les électrodes ECG, afin d'effectuer un électrocardiogramme.

Le dispositif électronique portatif peut être une montre électronique.

Dans un mode de réalisation, la lunette comprend un corps de lunette et la deuxième électrode ECG est formé par le corps de lunette, de sorte que l'intégralité du corps de lunette forme la deuxième électrode et que l'utilisateur peut toucher n'importe quelle portion du corps de lunette pour effectuer une mesure d'ECG.

Dans un mode de réalisation, la deuxième électrode ECG est électriquement connectée au module ECG quelle que soit la position angulaire de la lunette.

Dans un mode de réalisation, le dispositif comprend un connecteur électrique dans la carrure, configuré pour relier électriquement la lunette et le module ECG, le connecteur électrique assurant la connexion entre le module ECG et l'électrode.

Dans un mode de réalisation, le connecteur électrique est monté de manière amovible sur la carrure.

Dans un mode de réalisation, le connecteur électrique comprend un ressort en compression configuré pour assurer un contact électrique avec la lunette.

Dans un mode de réalisation, le ressort en compression est une lame et/ou le connecteur électrique comprend une pluralité de lames en compression, espacées angulairement.

Dans un mode de réalisation, le connecteur électrique comprend un anneau positionné autour de la glace et/ou d'un cadran du dispositif.

Dans un mode de réalisation, le connecteur comprend des pieds, et dans lequel les lames s'étendent d'un premier côté de l'anneau et les pieds s'étendent depuis un autre côté de l'anneau.

Dans un mode de réalisation, les pieds comprennent des plots et/ou des languettes.

Dans un mode de réalisation, les pieds sont électriquement connectés avec le module électronique ECG, de sorte que le signal ECG passe par au moins un desdits pieds.

Dans un mode de réalisation, le ressort en compression exerce une pression sur le pied de sorte à maintenir la connexion électrique du pied avec le module électronique ECG.

Dans un mode de réalisation, le connecteur électrique comprend un revêtement conducteur, comme de l'or.

Dans un mode de réalisation, la lunette comprend, sur une face interne, une série de crans aptes à interagir avec le ressort en compression, la série de cran et le ressort permettant de définir un déplacement pas à pas pour la rotation de la lunette.

Dans un mode de réalisation, la carrure comprend un support de lunette, la lunette étant montée à rotation sur le support de lunette et le connecteur électrique étant monté sur le support de lunette.

Dans un mode de réalisation, le support de lunette est électriquement connecté avec le module électronique ECG, de sorte que le signal ECG passe par le support de lunette.

Dans un mode de réalisation, la glace est montée sur la carrure ou sur la lunette.

Dans un mode de réalisation, la carrure comprend un corps principal, le support de lunette, et un joint d'étanchéité, de forme annulaire, entre le support de lunette et le corps principal de la carrure, le joint d'étanchéité permettant de maintenir en position le support de lunette et/ou d'isoler électriquement le support de lunette du corps principal de la carrure.

Dans un mode de réalisation, la lunette comprend des dentelures sur une face latérale externe, les dentelures faisant partie de la deuxième électrode.

Dans un mode de réalisation, le dispositif comprenant en outre un module de communication sans-fil, configuré pour communiquer de façon bidirectionnelle avec un terminal mobile, par exemple pour envoyer les résultats de l'ECG obtenus par le module ECG.

Dans un mode de réalisation, le dispositif électronique portatif est une montre, la montre étant par exemple une montre hybride avec des aiguilles mécaniques.

Dans un mode de réalisation, le fond de carrure comprend un capteur optique configuré pour émettre et recevoir de la lumière

Dans un mode de réalisation, la lunette est une pièce essentiellement de révolution.

Dans un mode de réalisation, la lunette est mobile en rotation antihoraire uniquement, notamment grâce au connecteur électrique et ses ressorts en compression.

Dans un mode de réalisation, le ressort travaille selon une direction parallèle à l'axe de rotation de la lunette ; dans un mode de réalisation, le ressort travaille selon une direction orthogonale à l'axe de rotation de la lunette.

La présente invention concerne également une méthode de prise de mesure d'un électrocardiogramme, ECG, à l'aide d'un dispositif tel que décrit précédemment, durant laquelle l'utilisateur met un bras en contact avec la première électrode et un autre bras en contact avec la deuxième électrode. On entend par « bras » le membre supérieur s'étendant de l'épaule à la main. En particulier, le dispositif est une montre-ECG, portée sur un poignet de sorte que la première électrode soit au contact dudit poignet et l'utilisateur touche la lunette rotative avec l'autre main.

La présente invention concerne aussi une utilisation d'un dispositif tel que précédemment décrit pour réaliser un électrocardiogramme.

### Brève description des dessins

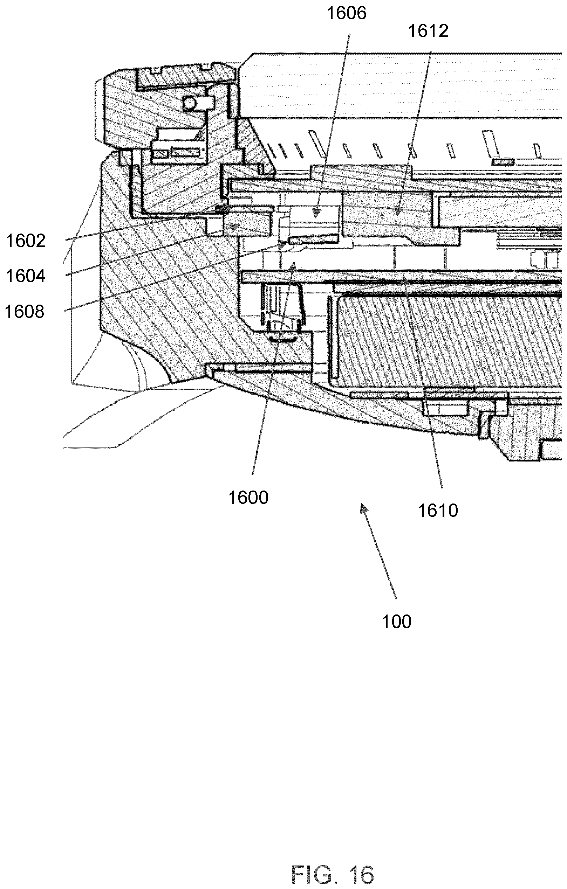

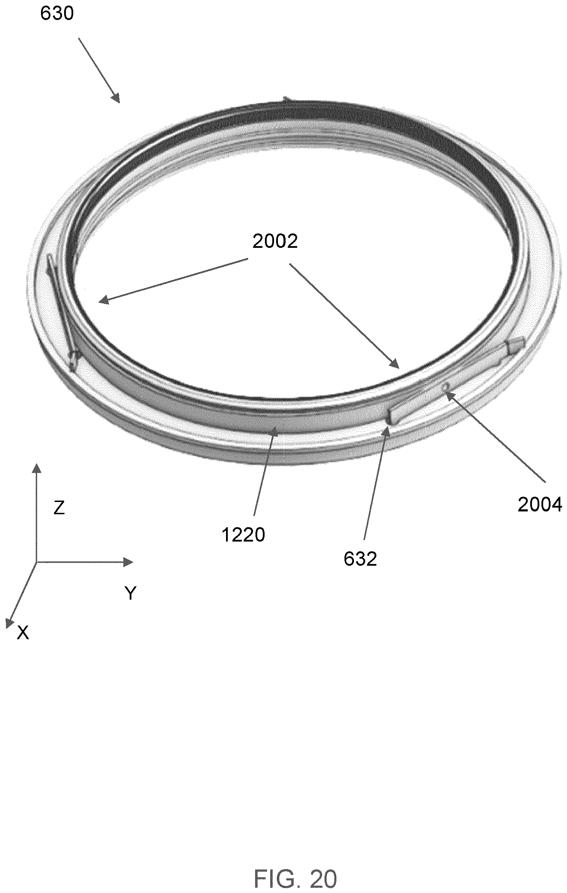

D'autres caractéristiques, détails et avantages apparaîtront à la lecture de la description détaillée ci-après, et à l'analyse des dessins annexés, sur lesquels :
**Fig. 1****.**
   Cette figure présente une vue du dessus (selon une direction Z) de la montre-ECG, où le cadran, la glace et la lunette en particulier sont visibles, selon un mode de réalisation (les aiguilles ne sont pas représentées).
**Fig. 2**
   Cette figure présente une vue du dessous (selon une direction Z) de la montre-ECG, où le fond de carrure et la carrure en particulier sont visibles, selon un mode de réalisation.
**Fig. 3**
   Cette figure présente une vue de côté (selon une direction X) de la montre-ECG, selon un mode de réalisation de montre-ECG hybride.
**Fig. 4**
   Cette figure présente une vue du dessus (selon une direction Z) de la montre-ECG, où la glace est visible, selon un mode de réalisation de montre non-hybride.
**Fig. 5**
   Cette figure présente une vue tridimensionnelle (partiellement transparente) de la montre-ECG, selon un mode de réalisation (lentille ou vitre de protection du capteur optique non illustrée).
**Fig. 6**
   Cette figure présente une vue en coupe selon le plan XZ d'une montre-ECG, selon un mode de réalisation.
**Fig. 7**
   Cette figure présente une vue tridimensionnelle et un agrandissement d'un connecteur électrique, selon un mode de réalisation.
**Fig. 8**
   Cette figure présente une vue tridimensionnelle et un agrandissement d'un connecteur électrique, selon un autre mode de réalisation.
**Fig. 9**
   Cette figure présente une vue tridimensionnelle de la lunette, avec la face inférieure visible.
**Fig. 10**
   Cette figure présente une vue en coupe, sur laquelle l'engagement du connecteur électrique avec les crans de la lunette est particulièrement visible.
**Fig. 11**
   Cette figure présente une vue tridimensionnelle, sur laquelle l'engagement du connecteur électrique avec les crans de la lunette est particulièrement visible.
**Fig. 12**
   Cette figure présente une vue tridimensionnelle d'un support de lunette.
**Fig. 13**
   Cette figure présente une vue en coupe d'une montre-ECG, selon un mode de réalisation dans lequel un pied du connecteur électrique est sous forme de plot.
**Fig. 14**
   Cette figure présente une vue en coupe d'une montre-ECG, selon un mode de réalisation où le connecteur électrique comprend un ressort et un roulement à bille.
**Fig. 15**
   Cette figure présente une vue tridimensionnelle d'un anneau de maintien entre la lunette et le support de lunette (non illustrés).
**Fig. 16**
   Cette figure présente une vue en coupe d'une montre-ECG, selon un mode de réalisation, où certains composants de la chaine électrique pour l'ECG sont particulièrement visibles.
**Fig. 17**
   Cette figure présente une autre vue en coupe d'une montre-ECG, à 90° par rapport à celle de la figure 16, selon un même mode de réalisation, où certains composants de la chaîne électrique pour l'ECG sont particulièrement visibles.
**Fig. 18**
   Cette figure présente un diagramme de la montre-ECG avec certains composants, notamment électroniques.
**Fig. 19**
   Cette figure présente des résultats de tests comparatifs avec la Withings ScanWatch 38mm, dont la lunette faisant office d'électrode ECG est fixe.
**Fig. 20**
   Cette figure présente une vue tridimensionnelle d'un support de lunette comprenant un autre mode de réalisation de connecteur électrique et de la lunette rotative.
**Fig. 21**
   Cette figure présente une vue détaillée du connecteur électrique de la Fig. 20 coopérant avec des crans de la lunette.

### Description des modes de réalisation

Des exemples de réalisation d'une montre-ECG selon la présente demande sont décrits dans cette section. Les exemples sont fournis pour illustrer et mieux comprendre les différents modes de réalisation.

La présente description concerne un dispositif électronique portatif comprenant un capteur d'électrocardiogramme (ci-après : capteur ECG). Dans un mode de réalisation particulier, qui est celui illustré, le dispositif électronique portatif est une montre (ci-après : montre-ECG). La montre-ECG peut comprendre un bracelet. Néanmoins, dans le cadre de la présente description, le terme montre-ECG n'inclut pas nécessairement le bracelet, qui est généralement fabriqué ailleurs et qui peut être assemblé aux endroits de vente.

Le dispositif électronique portatif est connecté, de sorte qu'il peut échanger des données à distance (sans-fil) de façon bidirectionnelle avec un terminal, de type smartphone. La connexion peut se faire par Bluetooth, comme un Bluetooth Low Energy (BLE). En particulier, les données échangées de la montre-ECG vers le terminal sont des données d'ECG acquises par la montre-ECG. La montre-ECG peut aussi recevoir des données par le terminal (données d'heure, d'alarme, notifications, etc.).

Dans un mode de réalisation, la montre-ECG est une montre hybride, c'est-à-dire une montre avec un cadran et des aiguilles pour indiquer les heures et les minutes.

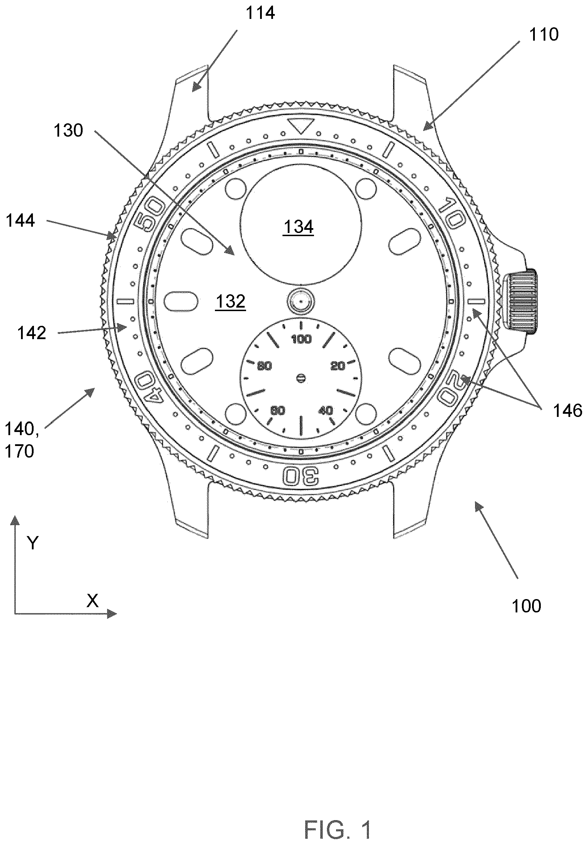

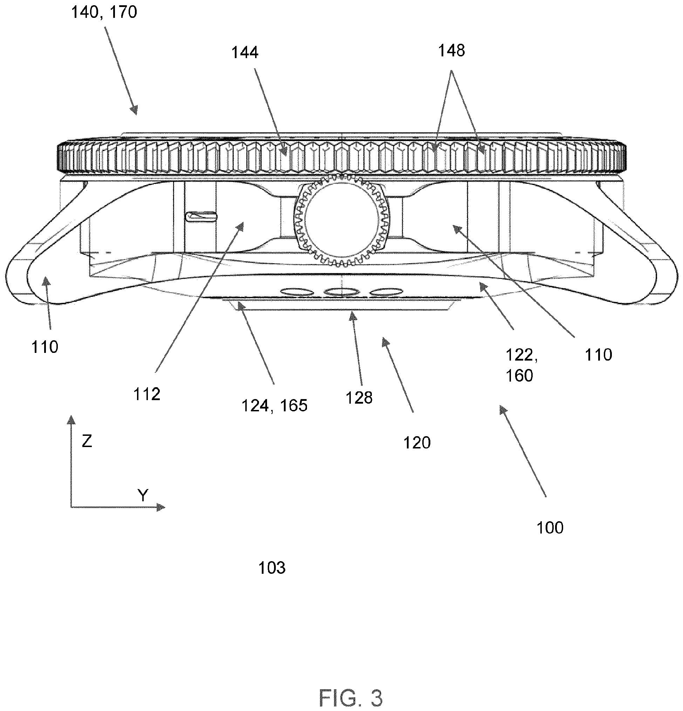

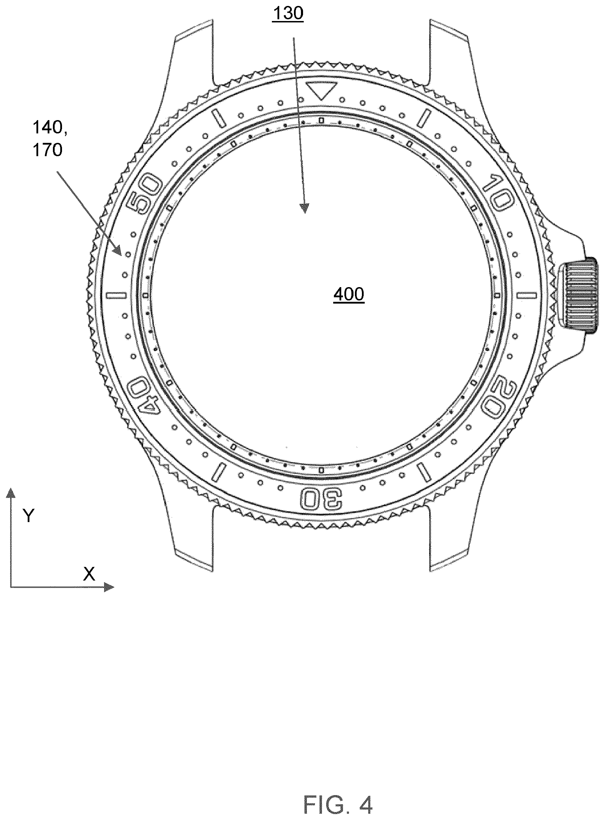

Les figures 1 à 3 illustrent une montre-ECG 100 électronique, de type hybride (avec un cadran, des aiguilles mécaniques, et éventuellement un écran intégré au cadran). La figure 4 illustre une montre-ECG 100, non hybride (sans aiguille mécanique mais avec un écran). Un repère normé (XYZ) est présenté sur ces figures. Dans la présente description, la notion de « dessus », « dessous » est défini par rapport à la direction Z, le dessus étant en direction de la glace et le dessous étant en direction du fond de carrure, qui vont être décrits ci-après.

La montre-ECG 100 peut comprendre une carrure 110 et un fond de carrure 120 qui est configuré pour être au moins partiellement au contact de la peau du poignet de l'utilisateur. La carrure 110 et le fond de carrure 120 sont solidaires entre eux. Dans un mode de réalisation, comme illustré sur les figures, la carrure 110 et le fond de carrure 120 sont deux pièces distinctes. Dans un mode de réalisation non illustré sur les figures, le fond de carrure 120 est une pièce intégrée à la carrure 110. La carrure 110 peut comprendre une paroi latérale 112, qui est généralement visible lorsque la montre-ECG 100 est portée au poignet. La carrure 110 peut comprendre des cornes 114 (deux paires, de part et d'autre de la carrure 110) pour attacher un bracelet (non représenté sur les figures). La carrure 110 peut inclure une pluralité de pièces.

La montre-ECG 100 peut aussi comprendre une glace 130 (« *glass* » ou « *crystal* » en anglais), généralement montée sur la carrure 110, de sorte que la glace 130 soit fixe. La glace 130 est ou peut comprendre un verre protecteur typiquement transparent et peut être réalisé en verre, en céramique, en plastique ou n'importe quel matériau transparent. Le contour de la glace 130 est ici typiquement de forme circulaire.

Dans le cas d'une montre hybride, sous la glace 130, la montre-ECG 100 comprend en outre un cadran 132 avec des aiguilles (des aiguilles physiques, qui ne sont pas représentées sur les figures). Le cadran 132 peut en outre recevoir un écran 134 (par exemple avec une ouverture dans le cadran qui permet de rendre visible un écran positionné juste sous le cadran), qui occupe par exemple un faible espace sous ou dans le cadran 132. Le glace 130 protège ces pièces et permet de les voir par transparence.

Dans le cas d'une « *smartwatch* » type Apple Watch, sous la glace 130, la montre-ECG comprend un écran 400 qui occupe une largeur proche de la largeur de la montre-ECG 100. Dans un mode de réalisation, l'écran 400 peut afficher des aiguilles. La glace 130 est alors le verre protecteur de l'écran 400.

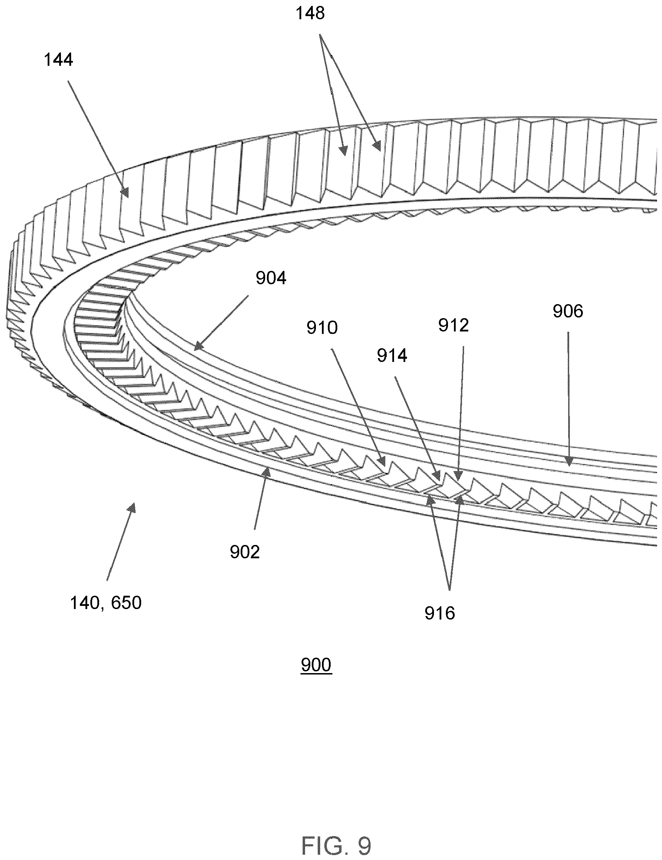

La montre-ECG 100 comprend en outre une lunette 140 (« *bezel* » en anglais), montée sur la carrure 110. La lunette 140 est positionnée autour de la glace 130 (radialement externe à la glace). La lunette 140 est montée à rotation par rapport à la carrure 110. La rotation peut se faire pas à pas (par exemple cran à cran). Le nombre de pas peut être de soixante ou de cent-vingt pas (qui correspondent donc angulairement à un ou deux crans par minute, une heure ayant 60 minutes qui représentent 360°). Toute valeur de pas peut être utilisée (par exemple des multiples de 15, 30 ou 60). L'utilisateur peut ainsi faire tourner la lunette 140, d'une façon classiquement connue sur une montre de type « DIVER ». La lunette 140 peut comprendre une face supérieure 142, une face inférieure (non visible sur les figures 1 à 3 mais qui sera décrite en relation avec la figure 9), une face latérale externe 144 et une face latérale interne (non visible sur les figures 1 à 3 mais qui sera décrite en relation avec la figure 9). La face supérieure 142 et la face latérale externe 144 sont visibles en conditions normales d'utilisation, que la montre-ECG 100 soit portée ou non au poignet.

La face supérieure 142 peut comprendre des marqueurs visuels 146, comme des graduations, des numéros, etc. Le but de ces marqueurs visuels 146 est d'assister l'utilisateur dans la gestion du temps. Par exemple, si, à un instant t, une personne souhaite savoir une durée qui va s'écouler, il suffit d'aligner un marqueur visuel 146 (par exemple une flèche ou un point qui indique le 0) avec les minutes. Par la suite, il suffit de regarder le marqueur visuel 146 vers lequel l'aiguille de minutes pointe pour connaître le temps écoulé depuis l'instant t. La face latérale externe 144 peut comprendre des dentelures 148 pour faciliter la préhension de la lunette par la main de l'utilisateur et donc la rotation de ladite lunette 140. Sur les montres « DIVER » traditionnelles, la lunette 140 permet à l'utilisateur d'estimer rapidement le temps de plongée écoulé.

La rotation de lunette 140 peut se faire dans le sens horaire, antihoraire ou les deux. Dans le cas d'une montre de type « DIVER » traditionnel, seul le sens antihoraire est permis, de sorte qu'une fausse manipulation ne fasse qu'augmenter le temps de plongée écoulé et non pas baisser ce temps.

La lunette 140 est une pièce de forme annulaire, essentiellement de révolution autour de la direction Z (à quelques modifications près). Plus de détails seront données par la suite.

Dans une variante non illustrée et moins fréquente sur les montres de type DIVER, la glace est montée sur la lunette rotative, de sorte que la glace se déplace avec la lunette.

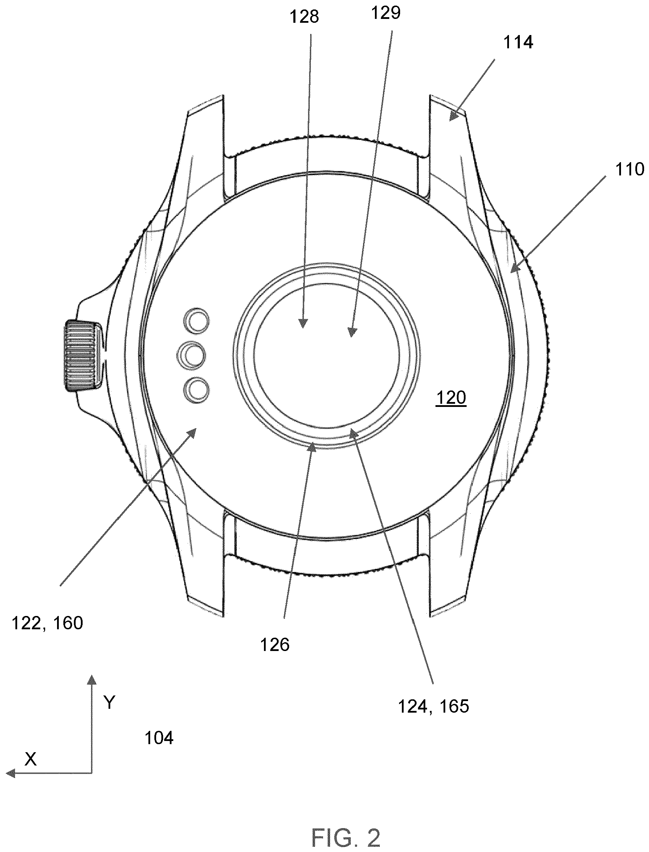

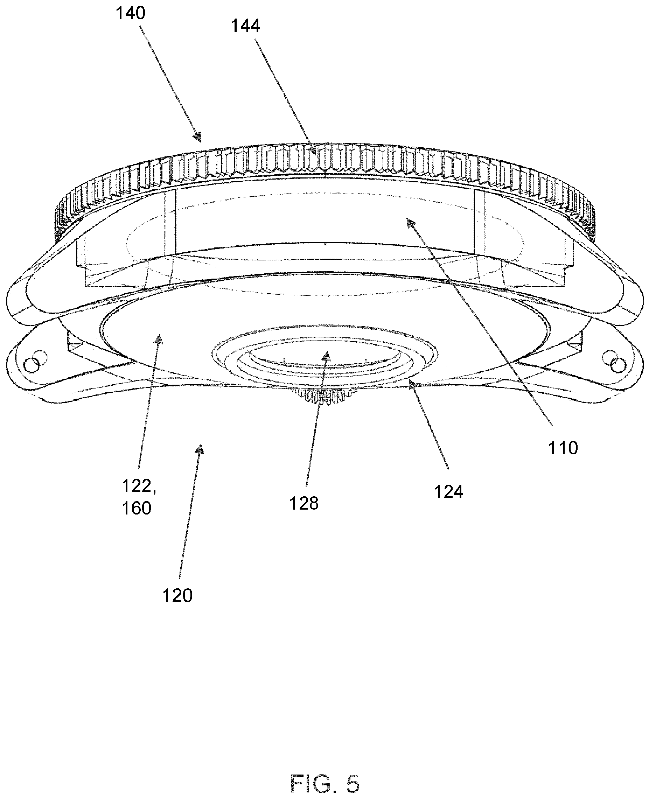

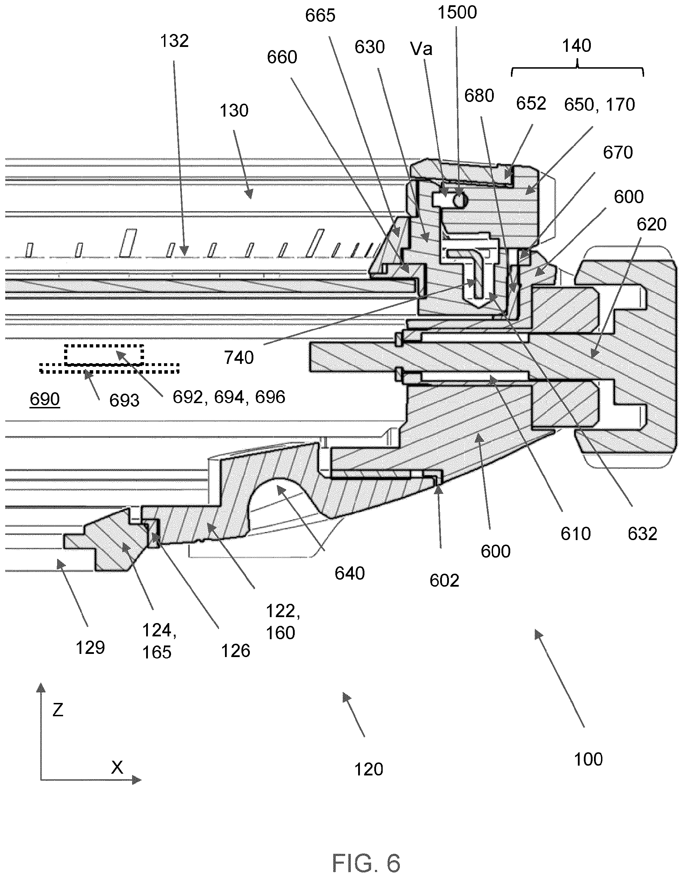

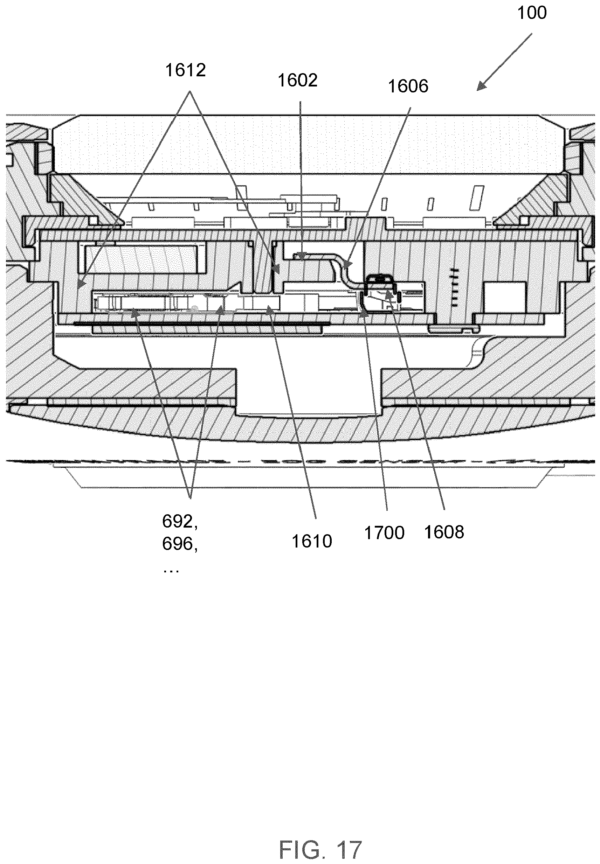

Pour récupérer les signaux électriques générés par le corps humain, la montre-ECG 100 comprend un capteur ECG. Le capteur ECG comprend notamment un ensemble d'électrodes (appelées électrodes ECG) et un module électronique ECG 692 (illustré très schématiquement sur la figure 6 mais mieux visible sur la figure 17), auquel les électrodes ECG sont électriquement connectées. Par électrode, il est signifié une pièce conductrice apte à recevoir un courant ou une tension électrique. La pièce peut être en matériau conducteur ou comprendre un revêtement conducteur. Par « conducteur », il est signifié « conducteur d'électricité ». Comme visible sur les figures 2, 3, 5, 6 une première électrode 160 se trouve sur le fond de carrure 120 afin d'être en contact avec la peau du poignet de l'utilisateur sur lequel ce dernier porte la montre-ECG 110. La première électrode 160 est électriquement connectée au module ECG 692.

Par exemple, le fond de carrure 120 peut comprendre un membre annulaire externe 122 et un membre annulaire interne 124 radialement à l'intérieur du membre annulaire externe 122. Le membre annulaire externe 122 est la première électrode 160. Les deux membres annulaires externes 122 et 124 peuvent être séparés par un joint 126. Le membre annulaire interne 124 peut entourer un capteur optique 128, avec au moins une LED, pour émettre de la lumière, et au moins une photodiode, pour recevoir de la lumière. Le membre annulaire interne 124 peut être en saillie (et présenter une forme frustro-conique). Le capteur optique 128 est typiquement un capteur PPG (photopléthysmographie). Le capteur optique 128 peut être positionné derrière une lentille 129, par exemple en verre, qui vient à l'interface de la peau du poignet. Le document PCT/EP2021/058955, au nom de Withings, et incorporé par référence, décrit en détail le capteur optique, qui se retrouve sur la Withings ScanWatch.

Comme visible sur les figures 1, 3, 4 à 6, une deuxième électrode ECG 170 se trouve sur la lunette 140 afin de pouvoir être facilement en contact avec la peau de l'autre main de l'utilisateur. La deuxième électrode 170 est elle-aussi électriquement connectée au module ECG 692.

Dans un mode de réalisation, la deuxième électrode 170 est la lunette 140, c'est-à-dire que l'intégralité de la lunette 140 ou une partie de la lunette (accessible par l'utilisateur) forme l'électrode 170. L'utilisateur peut ainsi toucher la lunette en n'importe quel endroit. Comparativement à une couronne faisant office d'électrode, la montre-ECG 100 peut être portée à la main gauche et la main droite indifféremment, sans que les gestes à effectuer pour effectuer un ECG soient les mêmes pour un utilisateur portant la montre à gauche ou à droite (symétrie d'utilisation), à l'inverse des couronnes-électrodes décrites en introduction. L'intégration d'une électrode sur une lunette rotative est contreintuitive du fait de la rotation de la lunette et dans la mesure où d'autres pièces de la montre semblent plus appropriées, comme la couronne. Néanmoins, les inventeurs se sont aperçus qu'il était possible d'obtenir des signaux de bonne qualité malgré les difficultés relatives à la chaîne électrique, qui sont générées par la mobilité relative entre la lunette et le boiter.

En particulier, la deuxième électrode 170 est connectée électriquement quelle que soit la position angulaire de la lunette rotative 140. L'utilisateur n'a donc pas besoin de mettre la lunette rotative 140 dans une position particulière pour effectuer un ECG. Comme indiqué précédemment, la rotation peut se faire par cran, dans lequel la lunette rotative 140 se place. Pour chaque cran, la connexion électrique peut se faire. Plus spécifiquement, y compris entre chaque cran, la connexion électrique peut aussi se faire. Ainsi, il y a une continuité totale de la connexion électrique entre la deuxième électrode 170 et le module ECG 692. En particulier, le geste à effectuer pour prendre un ECG est le même quelle que soit la position de la lunette rotative 140. Dit autrement, la deuxième électrode 170 est fonctionnellement invariante par rotation de la lunette rotative 140.

Les électrodes 160, 170 sont réalisées en matériau conducteur. Dans le mode de réalisation illustré sur les figures 1, 3, 4 à 6, les électrodes sont formées par les pièces elles-mêmes, qui sont en matériau conducteur. Alternativement, les électrodes, en particulier la première électrode, peuvent être formées par un revêtement conducteur déposé sur une surface (qui est elle-même conductrice ou non-conductrice).

La figure 6 illustre une vue en coupe de la montre-ECG 100 selon un mode de réalisation. La carrure 110 comprend notamment un corps principal 600 (ou corps de carrure), qui comprend la paroi latérale 112. Sur la figure 6, le corps principal 600 comprend une ouverture 610 au niveau de la paroi latérale 112 pour permettre la mise en place d'une couronne 620. La couronne 620 a ici un rôle uniquement d'interface utilisateur entre la montre-ECG 100 et l'utilisateur. A cette fin, la couronne 620 peut être mobile en rotation (ici autour de l'axe X) et/ou être mobile en translation (ici le long de l'axe X). La couronne 620 ne joue pas de rôle pour l'ECG et ne sera pas décrite plus en détail. Le fond de carrure 120 peut être monté sur le corps principal 600. Comme décrit précédemment, le fond de carrure 120 peut comprendre le membre annulaire interne 124, le membre annulaire externe 124, le joint 126 et la lentille 129. Un joint 602 peut être prévu entre le fond de carrure 120 et la carrure 110 (plus spécifiquement entre le membre annulaire externe 124 et le corps principal 600 de la carrure 110). Dans la vue en coupe de la figure 6, le membre annulaire externe 122 comprend un renfoncement 640, qui est un plot de positionnement pour une station de charge de batterie. La carrure 110 peut aussi comprendre un support de lunette 630, monté sur le corps principal 600. La lunette 140 est notamment montée sur le support de lunette 630, dans une position radialement externe. La glace 130 est elle aussi montée sur le support de lunette 630, dans une position radialement interne.

La lunette 140 peut comprendre un corps de lunette 650, qui est monté sur la carrure 110. Le corps de lunette 650 peut intégrer directement (par gravure ou autre) le décor traditionnel d'une montre, de sorte que la lunette 140 soit formée intégralement par le corps de lunette 650. Alternativement, comme illustré en figure 6, la lunette 140 comprend en outre un anneau de visualisation 652 (aussi appelé « décor ») qui peut être monté (par exemple par collage, par exemple avec de l'adhésif double face) sur une face supérieure du corps de lunette 650. Le décor comprend alors les marqueurs visuels 146 mentionnés précédemment. Le corps de lunette 650 est la deuxième électrode 170, de sorte que l'utilisateur peut toucher n'importe quelle partie du corps de lunette 650

(notamment les dentelures 148) pour effectuer un ECG. On entend par n'importe quelle partie du corps de lunette 650 n'importe quelle partie de la surface du corps de lunette 650 accessible à un utilisateur par contact.

Le corps de lunette 650 peut être en matériau conducteur, comme du métal (par exemple en acier inoxydable ou en alliage de titane). Alternativement, le corps de lunette 650 peut être en plastique chargé ou en céramiques conductrices. Alternativement encore, le corps de lunette 650 peut ne pas être conducteur et un revêtement conducteur est déposé sur tout ou partie du corps de lunette 650 (par exemple de la face supérieure ou de la face latérale externe jusqu'aux crans de la face inférieure).

Différents joints 660, 670, 680 sont disposés entre les différents composants pour assurer l'étanchéité, le maintien des pièces et/ou l'isolation électrique. Les joints 660, 670, 680 ont des formes annulaires ou circulaires. Ils sont de révolution autour de la direction Z. Un joint de cadran 660 permet d'assurer une isolation électrique entre le support de lunette 630, un rehaut 665 et le cadran 132. Un joint de protection 670, disposé entre le corps principal 600 de la carrure 110 et la lunette 140, permet de limiter l'introduction de poussière dans la montre-ECG 100. De plus, le joint de protection 670, en évitant que des débris ne viennent se coincer et créer une liaison électrique, concourt à une isolation électrique efficace entre la lunette 140 et le corps principal de carrure 600. Dans le cas où des débris viendraient se coincer entre la lunette et le corps principal 600 de carrure 110. Le joint de protection 670 permet aussi de faire office de butée mécanique pour la lunette 140 selon la direction en Z. Un joint d'étanchéité 680, disposé entre le support de lunette 630 et le corps principal 600 de la carrure 110, permet d'isoler électriquement le support de lunette 630 du corps principal 600 et de maintenir les pièces en position (résistance aux chocs notamment). De plus, le joint d'étanchéité 680 permet d'assurer une étanchéité à 10 atm, que le joint de protection 670 ne permet généralement pas (car il n'est pas un joint en compression). Le joint d'étanchéité 680 a typiquement une section en L (visible sur la figure 6). Sur les figures, les parois radialement externes du joint d'étanchéité 680 sont au contact d'une extension latérale et d'un plat du corps principal de la carrure et les parties radialement internes du joint d'étanchéité 680 sont au contact d'un angle du support de lunette 630.

La carrure 110 (et en particulier le fond de carrure 120, le cadran 132 et le corps principal 600 de la carrure 110) définit un volume interne 690 apte à recevoir différents composants, comme des composants électroniques. Ces composants électroniques sont ainsi protégés de l'eau ou de la poussière (avec les étanchéités appropriées, notamment permises par les joints précités).

Afin de permettre de prendre un ECG, la montre-ECG 100 comprend un module ECG 692 qui est logé dans le volume interne 690 (représenté schématiquement en pointillé sur la figure 6 et positionné schématiquement). Les deux électrodes 160, 170 sont électriquement connectées au module ECG 692. Le module ECG 692 est configuré pour récupérer des signaux électriques venant du corps humain et pour, après traitement, générer un électrocardiogramme. Le module ECG 692 peut être monté sur une carte électronique 693 (représenté schématiquement en pointillé sur la figure 6 et positionné schématiquement), de type circuit imprimé (« *printed circuit board* », PCB), où d'autres éléments de la montre-ECG sont aussi montés.

Le capteur optique 128 est connecté à un module PPG 694, lui-aussi positionné dans le volume interne, qui peut être lui-aussi monté sur la carte électronique 693 de la montre-ECG 100. Le module PPG 694 est configuré pour générer les consignes à destination des LEDs et pour récupérer les signaux électriques issues des photodiodes.

L'unité de contrôle 696 permet de piloter l'électronique embarqué sur la montre-ECG 100. L'unité de contrôle 696 peut par exemple inclure ou partiellement inclure le module ECG et le module PPG.

L'ensemble d'électrodes ECG peut comprendre une troisième électrode 165, par exemple sur le fond de carrure 120. En particulier, le membre annulaire interne 124 peut être la troisième électrode 165. Le document PCT/EP2021/058955, au nom de Withings, décrit l'agencement de la première et de la troisième électrode. La troisième électrode peut servir de référence aux deux autres électrodes. Alternativement, la première électrode 160 peut être le membre annulaire interne 124 et la troisième électrode, le cas échéant, la troisième électrode peut être le membre annulaire externe 122.

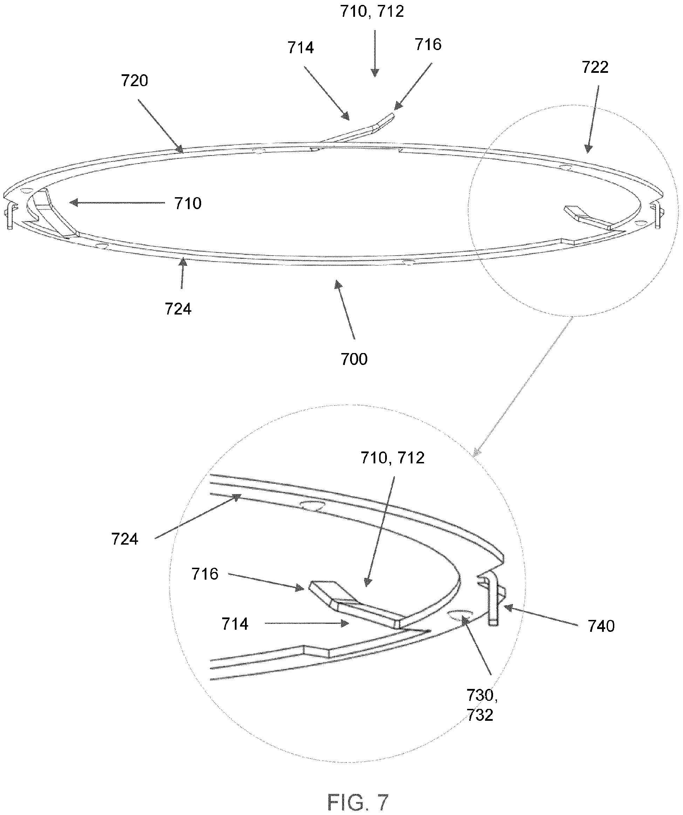

Afin d'assurer la connexion électrique entre le module ECG 692 et la deuxième électrode 170, la montre-ECG 100 (et en particulier la carrure 110) comprend un connecteur électrique 700 représenté en figures 7 à 8 notamment, qui assure la connexion électrique entre la lunette rotative 140 et le module ECG 692 (par exemple via une pièce de la carrure 110). Le connecteur électrique 700, étant monté sur la carrure 110, n'est pas en rotation, contrairement à la lunette rotative 140. Avantageusement, le connecteur électrique 700 est monté de manière amovible sur la carrure 110. En particulier, comme expliqué par la suite, le connecteur électrique 700 n'est pas soudé ou collé à la carrure 110 mais coopère simplement mécaniquement. Le connecteur électrique 700 est donc facilement remplaçable en cas d'usure.

Dans un mode de réalisation, le connecteur électrique 700 est réalisé dans un matériau conducteur, de sorte que l'intégralité du connecteur électrique 700 peut conduire le courant ou transmettre un potentiel.

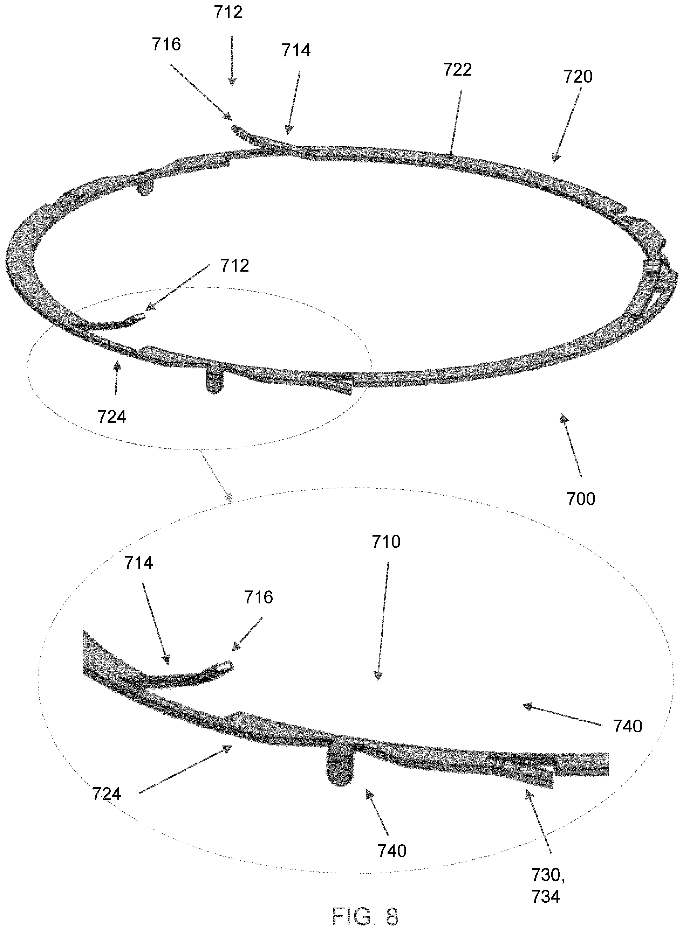

Le connecteur électrique 700 peut comprendre au moins un ressort 710, par exemple un ressort en compression. Dans le mode de réalisation illustré sur les figures 7 et 8 notamment, plusieurs ressorts en compression 710 sont prévus, répartis régulièrement (par exemple trois, répartis à 120° autour de la direction Z). Le ressort en compression 710 est notamment comprimé par la lunette 140, de sorte qu'à chaque rotation de cette dernière, le ressort en compression 710 permet de maintenir le contact au moins lorsque la lunette 140 est dans un pas (préférablement à tout instant). Le ressort en rotation 710 subit des frictions, des chocs répétés, de fatigues à la déformation. Lorsqu'une pluralité de ressorts en compression 710 est prévue, ceux-ci peuvent être mécaniquement synchronisés (tous dans le même état pour chaque position de la lunette) ou bien désynchronisés (certains plus ou moins comprimés). L'intérêt d'une pluralité de ressorts est d'assurer une redondance mécanique (et électrique dans notre cas), ce qui améliore la fiabilité de la montre-ECG.

Plusieurs modes de réalisations du ressort en compression 710 vont être décrit.

Comme illustré en figures 7 et 8, le ressort en compression 710 peut être une lame 712. La compression est assurée par la lunette 140 positionnée au-dessus (selon la direction Z) du connecteur électrique 700 et par le support de lunette 630 positionné en dessous (selon la direction Z), de sorte que le contact entre la lunette 140 et le connecteur électrique 600 est maintenu en permanence. Le connecteur électrique 700 comprend un anneau 720, configuré pour s'étendre autour (en projection en XY) de la glace 130 ou du cadran 132. L'anneau peut avoir la forme d'une lame plane. L'épaisseur de l'anneau peut être comprise entre de 0.05 mm et 1 mm, ou entre 0.1mm et 1mm, et préférablement entre 0,2 mm et 0,3 mm). Les ressorts 710 peuvent s'étendre depuis un premier côté 722 de l'anneau 720. Sur un deuxième côté 724 de l'anneau 720, peut se trouver un ou plusieurs pieds 730 permettant de maintenir stable le connecteur électrique 700 et d'assurer la conduction électrique. Afin de maximiser la planéité du connecteur électrique 600, l'anneau 720 est plan. De la même façon, sur le deuxième côté 724 peut se trouver une ou plusieurs pattes 740. Les pieds 730 et les pattes 740 seront décrits plus en détail par la suite. La redondance des contacts permet de maintenir un contact même si un des contacts venait à casser.

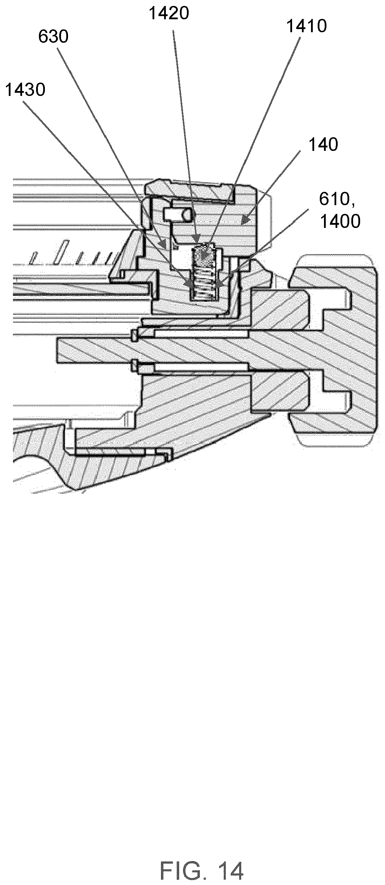

La figure 9 représente une vue isolée 900 de la lunette 140 et en particulier du corps principal 650 de la lunette 140. En particulier, la face latérale externe 144 et la face inférieure 902 y sont visibles. La face inférieure 902 fait face au connecteur électrique 700 et coopère mécaniquement avec ce dernier. En particulier, comme illustré, la lunette 140 comprend une série ou succession de crans 910 sur la face inférieure 902, qui participent au déplacement pas-à-pas de la lunette (un cran pour un pas). Les crans 910 peuvent s'étendre sur tout le tour de la face inférieure 900, de sorte qu'il n'y ait pas de zone morte lors de la rotation de la lunette 140. Les crans 910 sont engagés par le ressort 710 lorsqu'il est en compression (ou par une pièce poussée par le ressort). La fonction de cet engagement est double : la première est de bloquer (à tout le moins limiter) la rotation de la lunette 140, par exemple selon un système de cliquet ou de rochet (cran et lame), comme illustré en figures 9, 10 et 11, ou selon un système de came/suiveur (came lobée et roulement à bille par exemple, comme illustré en figure 14).

Selon la forme des crans 910, la rotation peut se faire dans le sens horaire ou antihoraire seulement. L'orientation de la lame 712 (qui s'étend depuis l'anneau dans un sens horaire ou anti-horaire - par rapport à la direction Z) peut définir le sens de rotation de la lunette 140. Une lame 712 s'étendant dans la direction anti-horaire peut bloquer la rotation en sens horaire ou inversement (sous réserve que le cran 910 soit conçu pour bloquer la rotation, comme décrit au paragraphe suivant).

Sur la face latérale interne 904 peut se trouver une rainure ou une gorge 906, configurée pour recevoir un anneau de maintien 1500 qui sera décrit par la suite.

Sur la figure 9, chaque cran 910 est formé par deux faces 912, 914. Les deux faces 912, 914 peuvent avoir des pentes égales, de sorte que le cran soit symétrique (non illustré). Deux crans successifs sont séparés par une arête 916. Si les faces 912, 914 ont une pente suffisamment raide, c'est l'orientation de la lame 712 qui définit le sens de rotation de la lunette 140. Alternativement, comme représenté sur les figures, la pente de la face 914 peut être plus raide (par exemple verticale ou quasiment verticale) que celle de la face 912, afin que la lame 712 vienne buter contre la face 914 d'un cran 910 pour bloquer la rotation dans un sens.

L'arête 916, qui sépare les deux faces 912, 914 peut présenter un méplat ou une forme arrondie pour limiter l'usure de la lame 712 à chaque rotation de la lunette 140.

Avec des crans 910 moins prononcés (pentes 912, 914 plus faibles) ou une lame 712 plus aplatie, il est possible d'avoir une lunette 140 montée à rotation dans le sens horaire et antihoraire, avec les crans qui définissent simplement des positions stables.

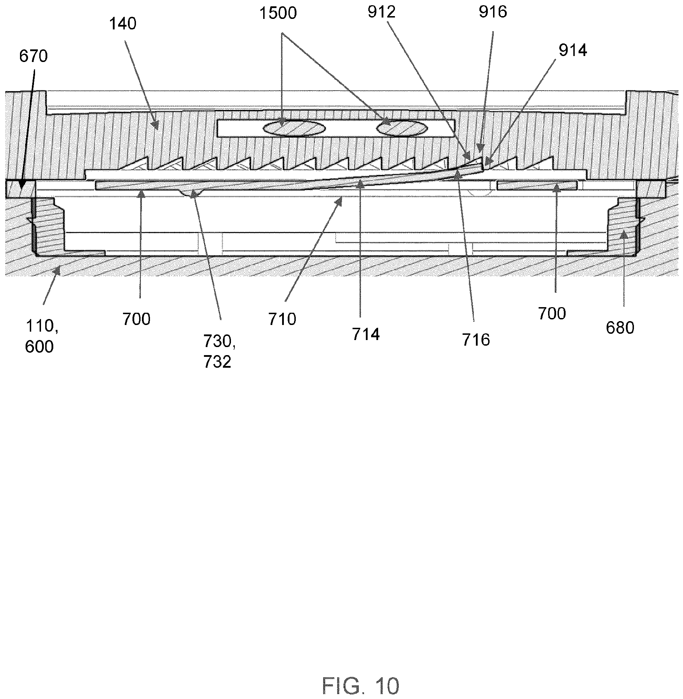

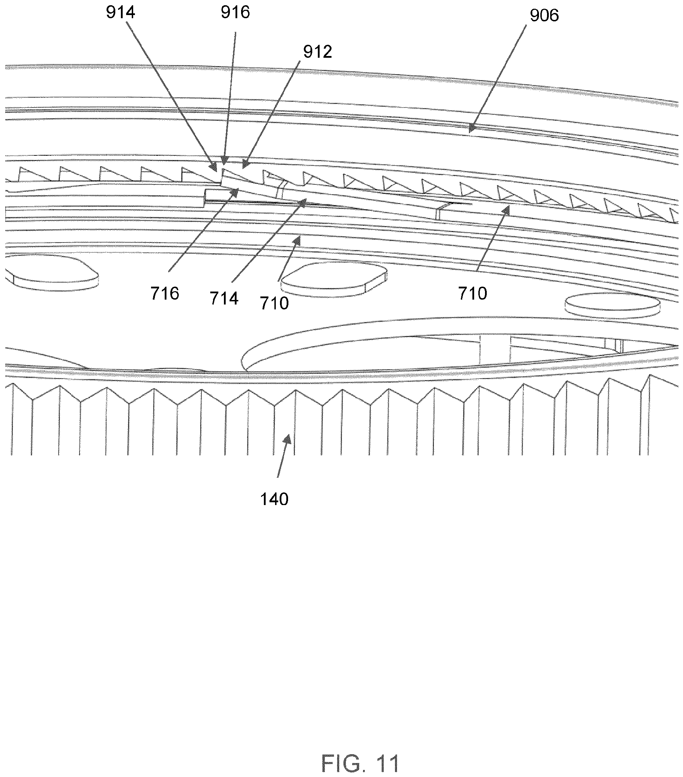

Comme visible sur les figures 7 et 8, 10 et 11, la lame 712 peut comprendre une première portion 714, qui s'étend depuis l'anneau 720 selon une première pente puis une deuxième portion 716, selon une deuxième pente plus forte que la première pente (au repos ou par rapport au plan de l'anneau 720 - qui coïncide en général avec un plan en XY), qui s'étend depuis l'extrémité de la première portion 714. Cette double inclinaison permet d'optimiser l'engagement entre les crans 910 de la lunette 140 et le connecteur électrique 700. En effet, comme visible sur les figures 10 et 11 représentants l'engagement de la lame 712 dans un cran 910 de la lunette 140, la lame 712 présente une longueur bien supérieure à la distance entre deux crans 910 successifs (longueur de la lame le long de la périphérie supérieure à au moins trois crans voire cinq, et par exemple comprise entre quatre crans et sept crans). La deuxième pente de la deuxième portion 716 permet aussi de faciliter le blocage de cette deuxième portion 716 de la lame 712 dans le cran 910. La deuxième portion 716 joue aussi un rôle dans la sensation haptique de rotation de la lunette 140. L'intérêt d'avoir une lame 712 s'étendant sur une pluralité de crans est de limiter le déplacement angulaire (l'angle de la première pente notamment) et la déformation de la lame 712 à chaque dépassement de cran par rotation de la lunette 140, et ainsi de garder le matériau dans un domaine de déformation élastique (indépendamment de la présence ou non des deux portions 714, 716). De plus, la longueur de la lame détermine aussi l'inclinaison globale et permet un meilleur blocage de la rotation en alignant l'effort de rotation avec la direction de la première portion de la lame 714. Ces optimisations permettent de limiter l'usure du connecteur électrique 700, dont le rôle de connexion électrique entre la deuxième électrode 170 et le module ECG 692 est primordial au bon fonctionnement de la mesure ECG même après plusieurs milliers de rotations de la lunette 140.

Dans un mode de réalisation, illustré sur les figures 10 et 11 notamment, la deuxième portion 716 de la lame 712 peut reposer au moins partiellement sur la pente 912 du cran (contact ponctuel, linéaire ou même surfacique si les pentes sont similaires), qui est la pente la plus faible, et vient en butée contre la pente 914 du même cran, qui est la pente la plus forte. Cette configuration permet d'avoir une première portion 714 avec une faible pente et donc d'être faiblement déplacée, comme expliqué précédemment.

Dans un mode de réalisation non illustré sur les figures, la lame présente une seule portion, droite ou courbée. Afin de garder une longueur de lame supérieure à plusieurs (trois ou cinq par exemple) crans tout en maintenant un engagement de la lame avec un cran, la profondeur des crans de la lunette peut être diminuée par rapport à la configuration avec la portion 716 plus inclinée que la portion 714.

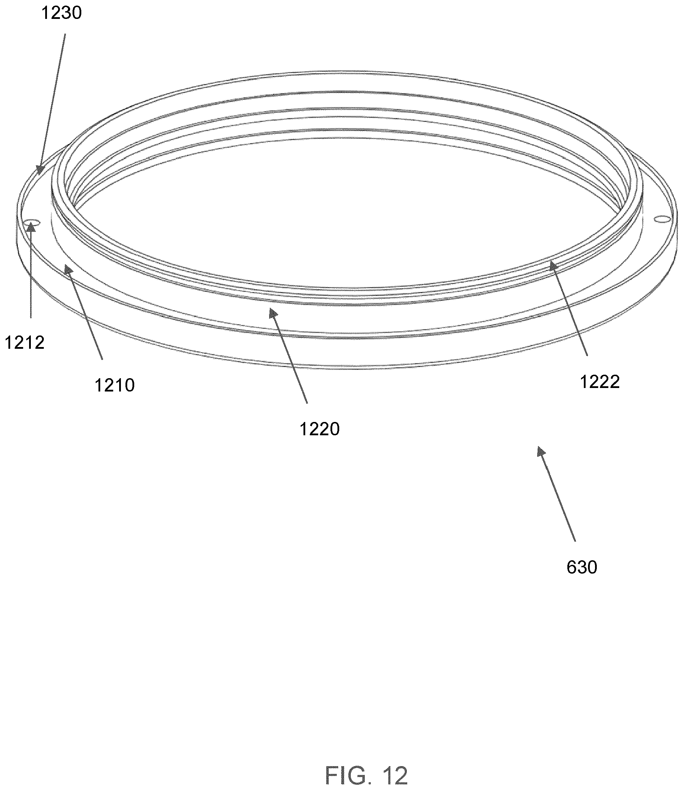

La carrure 110 peut en outre comprendre un support de lunette 630, introduit en relation avec la figure 6 et illustré plus en détail sur la figure 12. Le support de lunette 630 reçoit et porte notamment la lunette rotative 140. Faisant partie de la carrure 110, le support de lunette 630 est fixe et est configuré pour guider en rotation la lunette 140. Dans le mode de réalisation illustré, le support de lunette 630 comprend un anneau 1210, une paroi cylindrique interne 1220, s'étendant le long de la direction Z (vers les Z positifs typiquement) depuis un bord interne de l'anneau 1210, et une paroi cylindrique externe 1230, s'étendant depuis le long de la direction Z (vers les Z positifs typiquement) depuis un bord externe de l'anneau 1210. Radialement à l'intérieur de cette paroi cylindrique interne 1220 se trouve le cadran 132 (dans le cas d'une montre-ECG hybride). La paroi cylindrique interne 1220 peut comprendre une rainure 1222, sur une face externe de la paroi cylindrique interne 1220 (la face de la paroi cylindrique interne 1220 qui est tournée vers la paroi cylindrique externe 1230 donc), pour recevoir un anneau de maintien 1500 (représenté en figures 6, 13 et 15 notamment). La rainure 1222 du support de lunette 630 est en regard de la rainure 906 de la lunette 140 (visible en figures 6, 9, 11 et 12), définissant ainsi un volume annulaire Va à l'intérieur duquel se trouve l'anneau de maintien 1500. Cette pièce sera décrite par la suite. Le support de lunette 630, plus spécifiquement l'anneau 1210, peut aussi comprendre un ou plusieurs orifices de blocage 1212.

Comme indiqué précédemment, le support de lunette 630 repose sur le joint de maintien 680, visible en figures 6 et 10, qui interface le corps principal 600 de la carrure 110 et le support de lunette 630.

Le connecteur électrique 700 est positionné sur l'anneau 1210, entre la paroi cylindrique interne 1220 et la paroi cylindrique externe 1230. L'anneau 720 du connecteur électrique 700 est disposé parallèlement à l'anneau 1220 du support de lunette, à distance l'un de l'autre par la présence des pieds 730.

Le connecteur électrique 700 établit une connexion électrique entre la lunette 140 et le module ECG 692. En particulier, la connecteur électrique 700 est électriquement connecté avec le support de lunette 630 via notamment les pieds 730, qui font office de contacteurs électriques localisés. Par localisés, on entend que le contact électrique se fait à un endroit spécifiquement prévu par les concepteurs de la montre-ECG. Le fait de concentrer le contact sur une petite zone permet d'augmenter la force de contact entre le connecteur électrique 700 et le support de lunette 630. La résistance de contact en est réduite. La chaine électrique jouant un rôle primordial dans la qualité du signal ECG, de simple contacts métal-métal entre deux portions planes par exemple (par exemple l'anneau 720 du connecteur électrique 630 et l'anneau 1210 du support de lunette 680) peut générer du bruit. En utilisant des contacteurs électriques localisés (contact ponctuel ou linéaire, mais sur une distance de quelques millimètres maximum et précisément établie), la chaine électrique est plus stable.

La présence de plusieurs pieds 730 sur la deuxième côté 724 de l'anneau 720 du connecteur électrique 700 permet d'assurer une continuité électrique même si de micro-déplacements devaient intervenir. Les pieds 730 peuvent être répartis régulièrement le long de la périphérie du connecteur électrique 630. On peut prévoir entre deux et dix pieds (6 sur les figures 7 et 8).

Les pieds 730 permettent en outre d'assurer la stabilité mécanique du connecteur électrique 700 sur le support de lunette 630. A cet effet, au moins trois pieds 730 (typiquement espacés, par exemple espacés régulièrement) sont prévus. Par exemple, entre 3 et 10 pieds peuvent être prévus, ou encore entre 4 et 7 pieds, ou encore 6 pieds (répartis tous les 60 degrés). La lunette 140, quand elle est tournée par l'utilisateur, génère un effort sur le ou les ressorts 710, qui transmettent l'effort aux pieds 730. La stabilité mécanique du connecteur électrique 700 ainsi que la force générée par la compression des ressorts 710 concourt à la stabilité électrique de la montre-ECG. On prévoit typiquement autant de pieds 730 que de ressorts 710 en compression, pour symétriser au maximum le connecteur électrique 700.

Les figures 7, 10 et 13 illustrent un premier mode de réalisation des pieds 730, dans lequel les pieds 730 sont des plots 732. Les plots 732 peuvent avoir une forme partiellement sphérique ou hémisphérique ou plus généralement de forme arrondie. Alternativement, les plots peuvent avoir une extrémité légèrement plane. Dans un mode de réalisation, le plot 732 est solide (il ne se déforme pas sous les efforts impliqués par la lunette). Le plot 732 peut être dans le même matériau que le reste du connecteur électrique 700, le cas échéant avec le même revêtement.

La figure 8 illustre un deuxième mode de réalisation des pieds 730, dans lequel les pieds 730 sont des languettes 734 (ou des lames). Les languettes 734 peuvent avoir une forme de lame s'étendant de biais (strictement moins de 90°, par exemple en 10° et 45°) donc depuis l'anneau 720, du deuxième côté 724. Dans un mode de réalisation, les languettes 734 sont rigides, à tout le moins plus rigides ou nettement plus rigides que le ressort en compression 710, de sorte que la rotation de la lunette 140 comprime le ressort 710 et pas la languette 734. Dans un mode de réalisation, la languette 734 est légèrement déformable pour assurer le contact électrique. Dans un mode de réalisation, la languette 734 est comprimée à l'assemblage pour asseoir le connecteur électrique 700 sur le support de lunette 630.

Le support de lunette 630 est quant à lui électriquement connecté au module ECG 692 via des composants conducteurs, par exemple avec un revêtement conducteur comme de l'or (acier inoxydable plaqué or). Le support de lunette 630 peut être en matériau connecteur, comme en acier inoxydable (316L, 301, 304 ou 446), avec ou sans revêtement particulier (comme de l'or).

Comme visible en figures 6, 7, 8, le connecteur électrique 700 comprend aussi les pattes 740, déjà présentés en relation avec les figures 6 à 8. Les pattes 740 s'étendent depuis la deuxième côté 724 (côté opposé à celui des ressorts 710) de l'anneau 720. Le support de lunette 630 comprend l'orifice de blocage 632, qui est configuré pour recevoir la patte 740. Le connecteur électrique 630 est ainsi bloqué en rotation autour de l'axe Z et peut reprendre le couple que la lunette 140 exerce sur le connecteur électrique 600 lors de sa rotation ou lorsqu'elle est en butée contre le ressort 710. Pour ne pas nuire à la stabilité mécanique et à la connexion électrique via les pieds 730, la patte 740 est moins longue que la profondeur de l'orifice de blocage 632. Dans un mode de réalisation, au moins trois pattes 740 sont prévues, afin de symétriser le connecteur électrique 700 et de pouvoir palier à une déformation ou un endommagement d'une ou deux de celles-ci. Plus particulièrement, autant de pattes 740 que de pieds 730 que de ressorts en compression 710 sont prévus.

D'autres modes de réalisation du connecteur électrique vont être décrits. Dans un mode de réalisation illustré en figure 14, le connecteur électrique 630 comprend un ressort en compression 710 (par exemple un ressort hélicoïdal 1400) avec un roulement à bille 1410 (« *bail bearing* » en anglais). La lunette 140 comprend alors sur sa face interne une came 1420, par exemple lobée, sur laquelle roule le roulement à bille 1410. La connexion électrique se fait via le roulement à bille 1410 et éventuellement le ressort 710, 1400. Le ressort 710, 1400 peut être logé dans un orifice 1430 de la carrure 110, par exemple dans le support de lunette 630 (l'anneau 1210 du support de lunette 630, comme illustré) ou le corps principal de la carrure 600 directement, afin de maintenir le ressort en position. La forme de la came 1420 permet d'avoir une rotation pas à pas unidirectionnelle ou bidirectionnelle (horaire et/ou antihoraire). La forme de la came 1420 détermine aussi l'effort à appliquer pour entrainer la lunette 140 en rotation. Lorsque la lunette 140 se déplace, le roulement à bille 1410 roule sur la came 1420 de la lunette 140 et vient comprimer le ressort 610 selon la direction Z. Alternativement, le ressort 1400 peut être incliné. Cet arrangement permet notamment d'avoir une rotation unidirectionnelle. Le roulement à bille 1410 peut être recouverte d'un revêtement conducteur comme de l'or. Le ressort peut lui aussi être recouvert d'un revêtement conducteur comme de l'or. Alternativement, le ressort 1400 ne sert à qu'à gérer la rotation de la lunette et des lames 700, comme décrites précédemment, assurent la fonction électrique. Alternativement et inversement, le ressort 1400 ne sert qu'à gérer la connexion électrique et des lames 700, comme décrite précédemment, gère la rotation de la lunette.

Dans un mode de réalisation non illustré, le connecteur électrique 700 peut comprendre un balai (« *brush* » en anglais), configuré pour glisser sur la lunette rotative. Pour avoir une rotation cran à cran, un système de cliquet peut être mis en place, par exemple avec des lames similaires aux lames 710 ou un ressort hélicoïdal 1400 avec roulement à bille 1410. Le balai peut être électriquement relié au support de lunette. Le balai peut comprendre lui aussi un ressort en compression pour assurer le contact entre le balai et la lunette.

Le connecteur électrique 700 est formé en un matériau conducteur. Alternativement ou complémentaire, le connecteur électrique 700 est recouvert d'un matériau conducteur (revêtement conducteur). Le revêtement peut avoir une meilleure conductivité que le matériau du connecteur électrique 700. Dans un mode de réalisation, le connecteur électrique est en matériau conducteur (par exemple, en acier inoxydé, en phosphore-bronze, etc.) et les lames 710 ou tout le connecteur électrique 700 sont recouvertes d'un autre matériau conducteur (par exemple de l'or, du nickel, etc.). Dans un mode de réalisation, le connecteur électrique 700 est en métal comme de l'acier, par exemple de l'acier inoxydable, comme de l'acier 301 (acier résistant à la fatigue), ou du phosphore-bronze et est recouvert au moins partiellement et préférablement entièrement d'un revêtement, par exemple un revêtement métallique comme de l'or or du nickel. Alternativement, le connecteur 700 est en plastique avec un revêtement conducteur tel que mentionné précédemment. Le support de lunette 630 peut être réalisé dans un matériau identique au connecteur électrique 700.

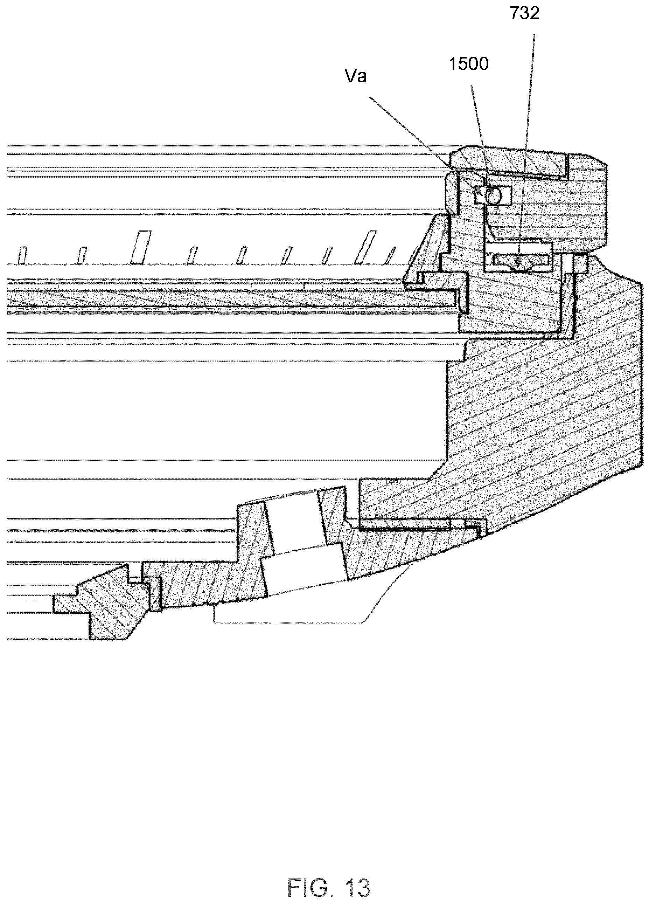

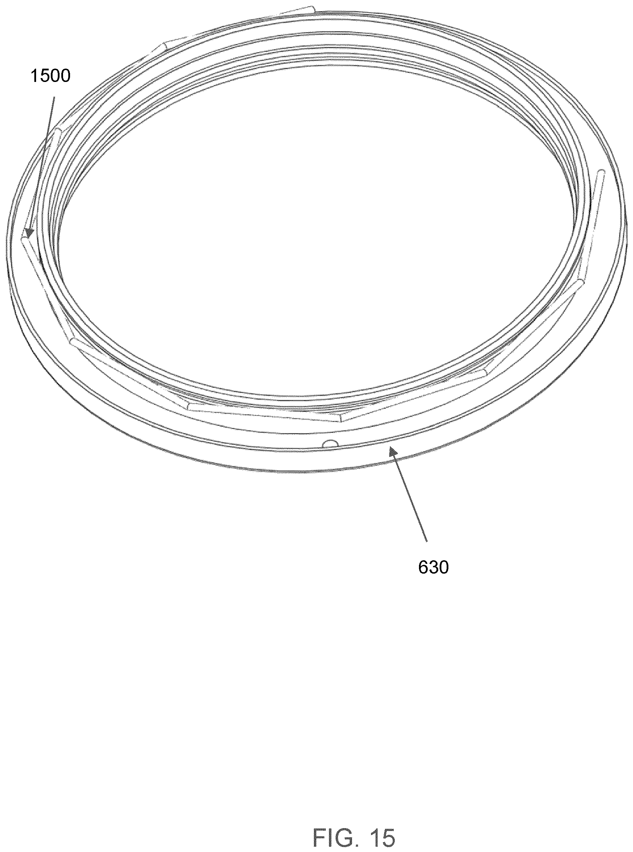

Comme mentionné auparavant, la montre-ECG comprend un anneau de maintien 1500, visible sur les figures 6, 10, 13, 14, 15, qui sert à bloquer le déplacement selon la direction Z de la lunette 140 par rapport à la carrure 110. L'anneau de maintien 1500, illustré sur la figure 15 (qui représente aussi le support de lunette 630), peut être une tige polygonale rigide s'étendant sur au moins 270 degrés dans le volume annulaire. Du fait de sa forme polygonale et non pas circulaire, l'anneau de maintien 1500 est logé, en fonction de la position angulaire le long du volume annulaire Va, alternativement dans la rainure 906 de la lunette 140 et dans la rainure 1222 du support de lunette 630, ce qui bloque le mouvement de la lunette 140 selon la direction en Z. Sur la figure 13, l'anneau de maintien 1500 est entre les deux ; sur la figure 6, l'anneau de maintien 1500 est au fond de la rainure 1222 de la lunette rotative 140. Du fait de sa forme polygonale, l'anneau de maintien 1500 n'occupe pas tout le volume annulaire Va, ce qui limite les frottements avec la lunette 140 lors de la rotation. L'anneau de maintien 1500 peut être légèrement comprimée lors de l'assemblage de la lunette 140 et peut rester légèrement comprimée une fois en position dans la volume annulaire Va.

Alternativement, un anneau de maintien s'étendant dans les deux rainures à la fois peut être prévu, mais la surface de frottement est augmentée par rapport au cerclage polygonal, ce qui peut gêner la rotation de la lunette.

Les dentelures 148 de la face latérale externe 144 de la lunette 140 (ou du corps de lunette 650), qui font ainsi partie de la deuxième électrode 170, permettent d'améliorer le contact électrique avec la peau de l'utilisateur (les dents pénètrent dans la peau, ce qui permet de diminuer la résistance de contact et d'avoir un effet de pointe au niveau des dentelures. Les performances de la montre-ECG peuvent ainsi être améliorées.

La montre-ECG 100 est typiquement une montre hybride, avec des aiguilles. Les aiguilles sont entrainées en rotation par un outre plusieurs micromoteurs, pilotés par l'unité de contrôle 696.

Comme décrit précédemment, la chaîne électrique par laquelle le signal électrique de l'ECG transite comprend l'électrode 170 de la lunette 140, puis le connecteur électrique 700, puis le support de lunette 630, puis un connecteur électrique intermédiaire 1600 (voir figure 16), puis un ressort 1700 (voir figure 17), puis la carte électronique 1610 (voir figure 16) sur laquelle est monté le module ECG 692 et/ou l'unité de contrôle 696. Le connecteur électrique intermédiaire 1600 sera décrit par la suite, en figures 16 et 17. Pour éviter les courts-circuits, la chaine de contact est isolée électriquement des pièces ne jouant pas de fonction électrique pour le capteur ECG (comme le corps principal 600 du la carrure 110 par exemple). Un membre porteur 1612 (« *holder member* ») est présent sur les figures 16 et 17, qui sert à maintenir en place certains composants de la montre ECG 100, et notamment le connecteur électrique intermédiaire 1600. Ce membre porteur 1612 est typiquement en plastique et peut avoir une forme de disque avec des empreintes pour recevoir les composants.

La chaine électrique entre le support de lunette 630 et le module ECG 692 peut comprendre donc le connecteur électrique intermédiaire 1600, illustré sur les figures 16 et 17, qui relie le support de lunette 630 à la carte électronique 693. Le connecteur intermédiaire 1600 peut comprendre un plateau 1602 (pièce plane, au contour en forme de banane), qui est en contact avec le support de lunette 630. Pour assurer le contact, une compression est instaurée lors de l'installation, avec une mousse 1604 positionnée sous le plateau 1602, qui est comprimée. La mousse 1604 permet en outre d'isoler électriquement. A partir du plateau 1602 s'étend un bras tridimensionnel coudé 1606 (dont la face en pente est visible sur la figure 16) qui va en direction du fond de carrure 120. A l'extrémité du bras 1606 se trouve un autre plateau 1608, parallèle au plateau 1602. Le plateau 1608 est en regard de la carte électronique, référencée 1610. La figure 17 illustre une vue à 90° de la figure 16. Le bras tridimensionnel coudé 1606 est visible, ainsi que la pente (mais vu de côté en coupe cette fois). Le plateau 1602 est hors de la vue en coupe. Un ressort 1700, en compression, est positionné entre le plateau 1608 et la carte électronique 1610 pour assurer le contact électrique. Enfin, l'on voit aussi sur la carte électronique 1610 différents composants, comme l'unité de contrôle 696 et/ou le module ECG 692, qui sont électriquement reliés au ressort 1700 par des circuits imprimés conducteurs.

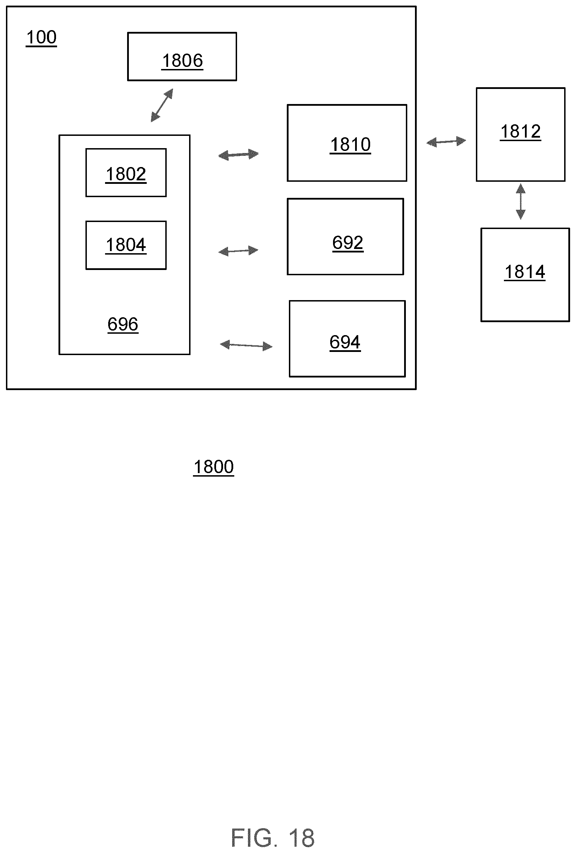

Comme représenté schématiquement en figure 18 qui illustre un diagramme 1800, l'unité de contrôle 696 peut comprendre un ou des processeurs 1802 et une mémoire 1804 qui stocke des instructions aptes à être exécutées par le processeur 1802.

La montre-ECG 100 peut également comprendre un accéléromètre 1806, relié à l'unité de contrôle 696 (pour le suivi de sommeil, d'activité, etc.).

Pour alimenter les différents composants en énergie électrique, la montre-ECG 100 comprend une batterie 1808, par exemple une pile ou une batterie rechargeable. Le renfoncement 640 décrit précédemment permet de placer le fond de carrure 120 sur une station de charge pour recharger la batterie 1808.

La montre-ECG 100 comprend un module de communication sans fil 1810, tel qu'un module Bluetooth ou Bluetooth Low Energy ou un module Wi-Fi ou un module cellulaire (GSM, 2G, 3G,4G, 5G, Sigfox, etc.), qui lui permet de communiquer de façon bidirectionnelle avec au moins un terminal externe 1612, tel qu'un téléphone portable. Le terminal externe 1812 peut ensuite communiquer (bidirectionnellement) avec un serveur distant 1814 pour le stockage et le traitement de données. Alternativement ou complémentairement, le module de communication sans fil 1810 peut communiquer directement avec le serveur distant 1814, par exemple via le réseau cellulaire ou via un réseau Wi-Fi. Les données obtenues par la montre-ECG 100, comme un électrocardiogramme, mais aussi les indications de rythme cardiaque, d'activité ou de saturation en oxygène, sont transmises au terminal externe 1812 via le module de communication sans fil 1810. L'unité de contrôle 696 peut traiter certains signaux avant de les envoyer, pour limiter la taille des données.

Le module PPG 694 et le module ECG 692 sont eux-aussi connectés à l'unité de contrôle 696 ou sont intégrés et/ou partiellement intégrés à celle-ci. Le module ECG 692 est connu en soi et ne sera pas décrit en détail. Différents types de composants électroniques peuvent être compris dans le module ECG 692 (processeur, résistance, condensateur, etc.).

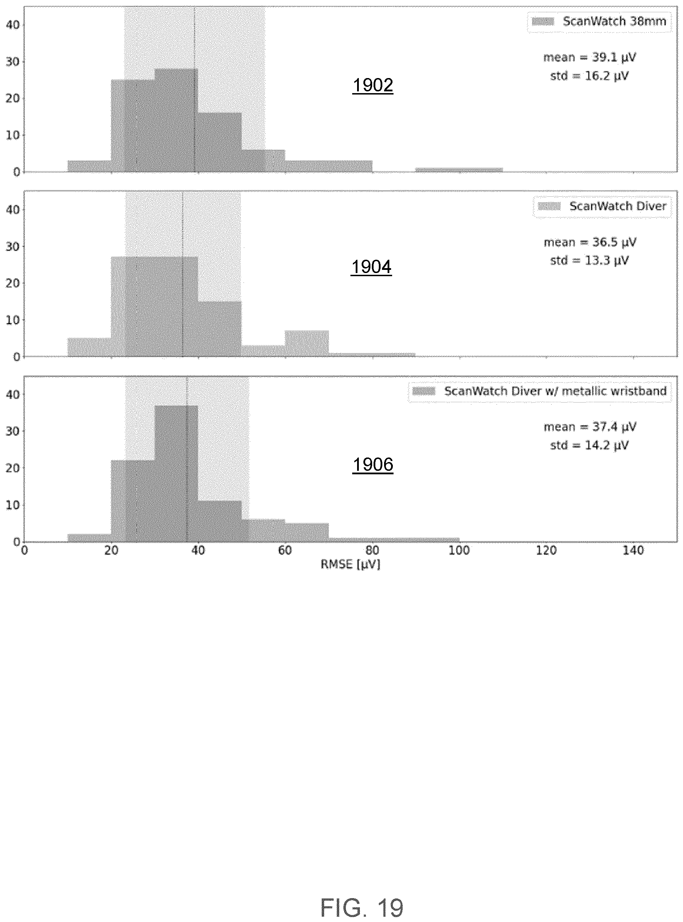

La figure 19 illustre des résultats de performance de la montre-ECG 100. Sur la figure 19, l'ordonnée des graphes représente le nombre d'enregistrements d'ECG (86 au total) fait avec une montre et l'abscisse représente l'erreur quadratique moyenne (« *mean squared error* », MSE, en anglais) d'une ScanWatch 38mm (graphe 1902), d'un montre-ECG 100 conforme au mode de réalisation des figures 1-3, 5-7, 9-15 avec un bracelet non conducteur (graphe 1904) et un bracelet métallique (graphe 1906), par rapport à un électrocardiogramme de référence (Schiller Cardiovit FT-1). « *Mean* » signifie la moyenne et « *std* » signifie la déviation standard. On observe que les résultats obtenus par la montre-ECG 100 ne sont pas moins bons que ceux obtenus par la ScanWatch 38mm, qui, en 2019, a obtenu la certification CE. Concernant les tests d'usure (deux mille rotations de la lunette), les résultats suivants ont été obtenus :

Pour le SNR (« *signal-noise ratio* », ou ratio signal sur bruit) :
- montres-ECG 100 non usées : 19,0 (±3,5) dB pour la montre-ECG 100 avec la lunette en acier et 19,2 (±3,4) dB pour la montre-ECG 100 avec la lunette en titane ;
- montres-ECG 100 usées : 17,8 (±3,6) dB pour la montre-ECG 100 avec la lunette en acier et de 18.0 (±3,5) dB pour la montre-ECG 100 avec la lunette en titane.

Pour le signal :
- montres-ECG 100 non usées : 725,9 (±261,6) dB pour la montre-ECG 100 avec la lunette en acier et 705,7 (±258,7) dB pour la montre-ECG 100 avec la lunette en titane ;
- montres-ECG 100 usées : 702,9 (±259,4) dB pour la montre-ECG 100 avec la lunette en acier et 732,5 (±283,6) dB pour la montre-ECG 100 avec la lunette en titane.

Pour le bruit :
- montres-ECG 100 non usées : 29,1 (±14,7) dB pour la montre-ECG 100 avec la lunette en acier et 27,4 (±11,0) dB pour la montre-ECG 100 avec la lunette en titane ;
- montres-ECG 100 usées : 32,9 (±10,9) dB pour la montre-ECG 100 avec la lunette en acier et de 31,7 (±10,1) dB pour la montre-ECG 100 avec la lunette en titane.

De plus, il a été observé qu'aucun bruit additionnel pouvant perturber l'analyse de l'ECG n'intervenait. Les résultats de test ont démontré que la montre-ECG 100 telle que présentée dans la description donnait des résultats d'ECG de qualité et que sa résistance à l'usure était bonne.

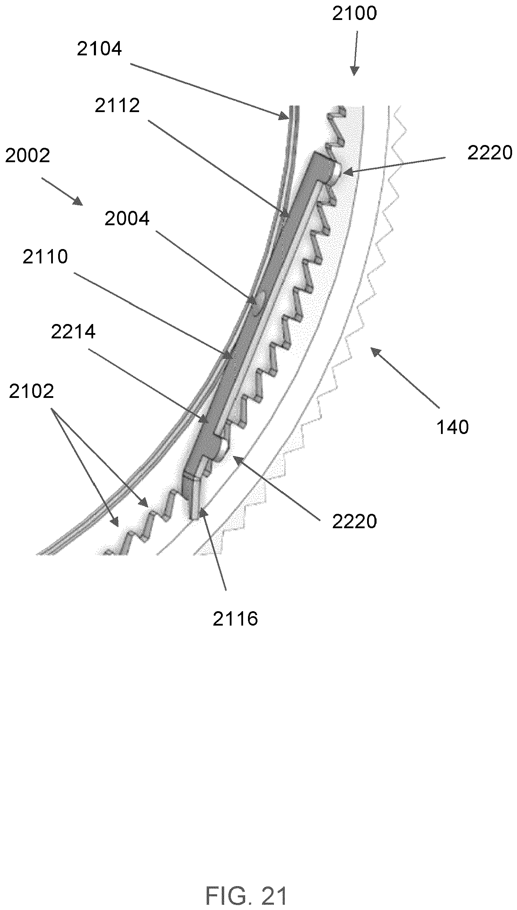

Un autre mode de réalisation du connecteur électrique et de la lunette rotative va être décrit par la suite, en référence aux figures 20 et 21.

Comme visible sur la figure 20, le connecteur électrique comprend un ressort en compression, qui est une lame 2002 en contact avec la paroi cylindrique interne 1220 du support de lunette 630. Avantageusement, le connecteur électrique comprend une pluralité de ressorts en compression sous forme chacun d'une lame 2002 en compression. Les lames 2002 sont espacées angulairement, avantageusement régulièrement. Ici, le connecteur électrique 700 comprend trois lames 2002 espacées d'environ 120° entre elles. Dans le mode de réalisation illustré, le connecteur électrique est constitué de plusieurs ressorts en compression indépendants. Alternativement, le connecteur électrique peut comprendre un anneau qui relie la pluralité de ressorts en compression.

Chaque lame 2002 comprend un pied 2004, davantage visible sur la figure 21, permettant de maintenir stable le connecteur électrique et d'assurer la conduction électrique avec le support de lunette 630. En particulier, chaque pied 2004 est propre à coopérer avec la paroi cylindrique interne 1220 du support de lunette 630.

Comme illustré sur la figure 21, la lunette 2100 comprend une série ou succession de crans 2102 arrangés sur la face latérale interne 2104, qui participent au déplacement pas-à-pas de la lunette (un cran pour un pas). Les crans 2102 peuvent s'étendre sur tout le tour de la face latérale interne 2102, de sorte qu'il n'y ait pas de zone morte lors de la rotation de la lunette 2100.

Les lames 2002 sont propres à s'engager dans les crans 2102. A cet effet, chaque lame 2002 comprend une première partie 2110 et une deuxième partie 2112 s'étendant de part et d'autre du pied 2004, les deux parties s'étendant sensiblement tangentiellement à la paroi cylindrique interne 1220. Les deux parties s'étendent par exemple selon une longueur semblable de part et d'autre du pied 730.

La première partie 2110 peut comprendre une première portion 2114 (semblable à la première portion 714), qui s'étend depuis le pied 2004 selon une première pente puis une deuxième portion 2116 (semblable à la deuxième portion 716), selon une deuxième pente plus forte que la première pente (au repos ou par rapport paroi cylindrique interne 1220), qui s'étend depuis l'extrémité de la première portion 2114 et propre à s'engager dans un cran 2102, comme visible sur la figure 21.

Chaque lame 2002 peut comprend au moins une patte 2220 s'étendant depuis un rebord de la lame 2002, orthogonalement au plan XY. Ici deux pattes 2220 sont prévues, une lame s'étendant depuis chaque partie 2110, 2112. Chaque patte 740 est propre à s'insérer dans un orifice de blocage 632 arrangé dans le support de lunette 630 (voir figure 20). Le connecteur électrique 700 est ainsi bloqué en rotation autour de l'axe Z et peut reprendre le couple que la lunette 140 exerce sur le connecteur électrique 700 lors de sa rotation ou lorsqu'elle est en butée contre le ressort 710.

Dans le mode de réalisation des figures 20 et 21, le ressort en compression travaille dans une direction transverse à l'axe Z, et plus particulièrement dans une direction du plan XY, ce qui permet d'être indépendant des tolérances d'assemblage selon l'axe Z. Les tolérances d'assemblage selon l'axe Z peuvent mener à des variances d'assemblage et pourraient, *in fine,* affecter la qualité des signaux ECG. Dans le mode de réalisation des figures 7 et 8, le ressort en compression travaille parallèlement à l'axe Z.

## Revendications

1. Dispositif électronique portatif (100), configuré pour être positionné sur un poignet d'un utilisateur, le dispositif portatif (100) permettant d'effectuer un électrocardiogramme, ECG, ledit dispositif (100) comportant :
- une carrure (110),
- un fond de carrure (120), configuré pour être au moins partiellement en contact avec la peau du poignet,
- une glace (130),
- une lunette (140), montée sur la carrure (110) et entourant la glace (130), mobile en rotation par rapport à la carrure (110),
- une première électrode ECG (160), en matériau conducteur, sur le fond de carrure (120) et configurée pour être en contact avec la peau du poignet,
- une deuxième électrode ECG (170), en matériau conducteur, sur la lunette (140),
- un module électronique ECG (692), électriquement connecté à la première électrode ECG (160) et la deuxième électrode ECG (170), et configuré pour recevoir et traiter des signaux électriques venant d'un utilisateur et récupérés par les électrodes ECG, afin d'effectuer un électrocardiogramme,
dans lequel la lunette (140) comprend un corps de lunette (650) et la deuxième électrode ECG est formée par le corps de lunette (650), de sorte que l'intégralité du corps de lunette (650) forme la deuxième électrode et que l'utilisateur peut toucher n'importe quelle portion du corps de lunette (650) pour effectuer une mesure d'ECG.

2. Dispositif selon la revendication 1, dans lequel la deuxième électrode ECG (170) est électriquement connectée au module ECG (150) quelle que soit la position angulaire de la lunette (140).

3. Dispositif selon l'une quelconque des revendication 1 à 2, comprenant un connecteur électrique (700) dans la carrure (110), configuré pour relier électriquement la lunette (140) et le module ECG, le connecteur électrique (700) assurant la connexion entre le module ECG et la deuxième électrode (170), dans lequel le connecteur électrique (700) comprend au moins un ressort en compression (710) configuré pour assurer un contact électrique avec la lunette.

4. Dispositif selon la revendication 3, dans lequel le connecteur électrique (700) est monté de manière amovible sur la carrure (110).

5. Dispositif selon la revendication 3 ou 4, dans lequel l'au moins un ressort en compression (710) est une lame (712, 2012).

6. Dispositif selon l'une quelconque des revendications 3 à 5, dans lequel le connecteur électrique (700) comprend une pluralité de ressorts en compression (710), les ressorts en compression étant espacées angulairement.

7. Dispositif selon l'une quelconque des revendications 3 à 6, dans lequel le connecteur (700) comprend des pieds (730), par exemple des plots (732) et/ou des languettes (734), dans lequel les pieds (730) sont électriquement connectés avec le module électronique ECG, de sorte que le signal ECG passe par au moins un desdits pieds.

8. Dispositif selon la revendication 7, dans lequel chaque ressort en compression (710) exerce une pression sur les pieds (730) de sorte à maintenir la connexion électrique des pieds avec le module électronique ECG (692).

9. Dispositif selon la revendication 7 ou 8, dans lequel le dispositif comprend entre 3 et 10 pieds (730), notamment entre 4 et 7 pieds.

10. Dispositif selon l'une quelconque des revendications 7 à 9, dans lequel le connecteur électrique (700) comprend un anneau (720) positionné autour de la glace (130), dans lequel l'au moins un ressort en compression (710) s'étend d'un premier côté de l'anneau (720) et les pieds (730) s'étendent depuis un autre côté de l'anneau (720).

11. Dispositif selon l'une quelconque des revendications 7 à 10, dans lequel les pieds (730) sont des languettes (734), les languettes (734) étant plus rigides que l'au moins un ressort en compression (710).

12. Dispositif selon l'une quelconque des revendications 3 à 11, dans lequel l'au moins un ressort en compression (710) travaille parallèlement à un axe de rotation (Z) de la lunette.

13. Dispositif selon l'une quelconque des revendications 3 à 11, dans lequel l'au moins un ressort en compression (710) travaille orthogonalement à un axe de rotation (Z) de la lunette.

14. Dispositif selon l'une quelconque des revendications 3 à 13, dans lequel la lunette (140) comprend, sur une face interne, une série de crans (910) aptes à interagir avec l'au moins un ressort en compression (710), la série de crans et le ressort permettant de définir un déplacement pas à pas pour la rotation de la lunette (140).

15. Dispositif selon l'une quelconque des revendications 3 à 14, dans lequel le connecteur électrique (700) comprend un revêtement conducteur, comme de l'or.
